(19) Europäisches Patentamt
European Patent Office
Office européen des brevets

(11) **EP 2 390 646 A1**

(12) **EUROPEAN PATENT APPLICATION**

(43) Date of publication:
**30.11.2011 Bulletin 2011/48**

(51) Int Cl.:
**G01N 1/30** *(2006.01)* **C12Q 1/68** *(2006.01)*

(21) Application number: **10164129.8**

(22) Date of filing: **27.05.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS**<br><br>(71) Applicant: **Biocartis SA**<br>**1024 Ecublens (CH)** | (72) Inventors:<br>• **Offermans, Paulus H. G.**<br>  **5600 AE Eindhoven (NL)**<br>• **Pierik, Anke**<br>  **5600 AE Eindhoven (NL)**<br>• **Meijering, Bernadet D. M.**<br>  **5600 AE Eindhoven (NL)**<br>• **Bakker, Frederikus**<br>  **5600 AE Eindhoven (NL)** |

(54) **Prevention of crystal formation in liquid solutions during storage by adding a betaine**

(57) The present invention relates to the field of improving and stabilizing concentrated solutions and in particular to improving and stabilizing concentrated solutions for the extraction of nucleic acids from sample material. Thus, the present invention, further, relates to the field of molecular biology. The present invention describes the use of betaines for stabilizing organic salts or chaotropic agents in solution which is particularly useful in cases where organic salts or chaotropic agents are present at high concentration such as for example chaotropic agents in buffers for the extraction of nucleic acids from sample material. The present invention describes the corresponding buffers and further relates to processes for the extraction of nucleic acids from sample material as well as processes for the analysis of sample material. Moreover, the present invention relates to kits and cartridges comprising at least one container with the corresponding buffer.

EP 2 390 646 A1

## Description

FIELD OF THE INVENTION

The present invention relates to the field of improving and stabilizing solutions. In particular, the present invention relates to improving and stabilizing solutions for the lysis and extraction of nucleic acids from sample material. Some of these solutions, thus, find application in the field of molecular biology, i.e. for example, in the process of preparing nucleic acids for amplification. The present invention therefore further relates to the field of molecular biology.

BACKGROUND OF THE INVENTION

Nearly all laboratory procedures performed today require the use and storage of several and sometimes even large numbers of solutions of various chemical compounds and mixtures. Routinely, such solutions are kept refrigerated in the laboratory in order to prevent degradative processes and to suppress potential bacterial or fungal growth. As a result of the reduced temperature in the refrigerator, or alternatively, temperature variation in the laboratory itself, some of the chemical compounds in these solutions may form crystals that precipitate out of these solutions. Before using any such solution, therefore laboratory personnel has to re-dissolve the precipitated crystals in order to obtain a solution with the original concentration. This process of re-dissolving precipitates can be very tedious and time-consuming. In particular, this is the case for concentrated solutions, e.g. solutions of chaotropic agents, because often and especially in the field of molecular biology such agents have to be used at high concentrations and are thus prone to precipitation.
Therefore, there is a need in the art to find suitable additives that allow to stabilize compounds in solution. Such additives would also be beneficial for commercial providers of pre-packaged solutions which are intended for immediate use by the customer as any such pre-packaged product would be significantly less appealing or possibly even unusable if laborious re-dissolution would be necessary.
A number of solutions for the lysis and extraction of nucleic acids from sample material contain high concentrations of chaotropic agents. For a wide range of applications lysis and extraction are followed by nucleic acid amplification, e.g. for analytical or diagnostic purposes. Frequently, however, the use of chaotropic agents in lysis and extraction solutions inhibits and/or disturbs the subsequent process of nucleic acid amplification. Therefore, there is, further, a need in the art to provide solutions containing chaotropic agents for the lysis and extraction of nucleic acids from sample material that can be used in lysis and extraction protocols followed by nucleic acid amplification, with good yields for nucleic acid extraction and minimal inhibition and/or disturbance of the nucleic acid amplification.

SUMMARY OF THE INVENTION

A large amount of laboratory work today is related to the isolation of nucleic acids from sample material in order to amplify and/or analyze these nucleic acids. In many cases a chaotropic agent is used in order to perform this isolation. Therefore, the corresponding solutions of chaotropic agents must be prepared and stored in a large number of laboratories today. Due to their mechanism of action chaotropic agents have to be applied at high concentrations (e.g. close to saturation). These concentrated solutions, however, naturally are prone to precipitation, e.g. as a result of temperature variation. Re-dissolving of precipitates sometimes poses a problem as it can be very tedious and time-consuming.
The present invention provides a solution to this problem by providing betaines that are effective in stabilizing solutions of organic salts and chaotropic agents. In particular, it has been found that suitable selection of the concentrations of betaine additive and chaotropic agent allows their use in buffers that can be applied for the isolation of nucleic acids from sample material with minimal or no interference with subsequent nucleic acid amplification reactions, e.g. by polymerase chain reaction (PCR).
Furthermore, as a result of the stabilization, the betaine additives allow to obtain concentrations of organic salts and chaotropic agents in solutions that exceed the saturation concentrations at a given temperature of solutions without betaine additive.
The use of betaine additives according to the present invention, thus allows the manufacture of highly concentrated solutions of organic salts and chaotropic agents that will not precipitate if stored in a refrigerator, i.e. at temperatures of 2 °C to 8 °C. Significantly, this renders unnecessary any assessment of the presence of precipitate in these solutions prior to use. Therefore, solutions stabilized by betaines according to the invention can be packaged into containers that do not allow to assess the presence of precipitate from the outside or which would not allow to take measures suitable to re-dissolve such precipitate.
Moreover, the present invention relates to improved lysis and extraction solutions, i.e. solutions for the lysis and extraction of nucleic acids comprising chaotropic agents and betaine additives, wherein the use of these solutions in the course of lysis and extraction protocols results in higher yields of extracted nucleic acids and/or in less inhibition and/or disturbance of subsequent nucleic acid amplification reactions performed with the extracted nucleic acids than the use of lysis and extraction solutions containing no such betaine additives. Such improved solutions for the lysis and extraction of nucleic acids can in some cases also be stabilized with respect to precipitation as discussed above, i.e. for example, be stable to precipitation if stored in a refrigerator, i.e. at temperatures of 2 °C to 8 °C, however, in other cases such solutions are not stable to precipitation under refrigerated conditions. Correspondingly, the solutions according to the present invention that are stabilized with respect to precipitation as discussed above can in some cases also be improved solutions for the lysis and extraction of nucleic acids as discussed above.

Furthermore, the present invention is directed at processes for the extraction of nucleic acids from sample material using the buffers of the invention as well as to processes for the analysis of sample material, e.g. by PCR, employing the buffers of the present invention.

The use of betaines for the improvement and stabilization of solutions according to the invention comprises aqueous solutions as well as non-aqueous solutions. The stabilization effect, however, is particularly pronounced for organic salts, i.e. salts of organic cations, and chaotropic agents in aqueous solution.

BRIEF DESCRIPTION OF THE DRAWINGS

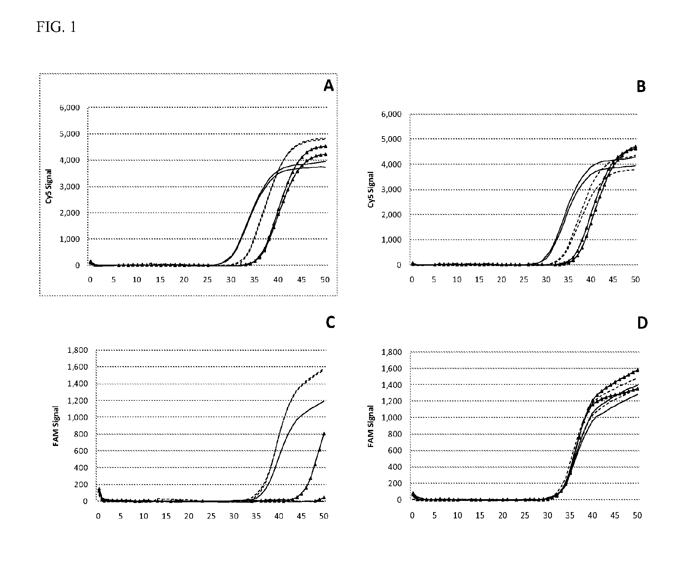

Fig. 1 PCR analysis of nucleic acid extracted from a human feces sample spiked with *Clostridium difficile* genomic DNA and *Bacillus subtilis* comK plasmid DNA using a lysis buffer without betaine (Lysis Buffer A) and a lysis buffer supplemented with 1.0 M betaine (Lysis Buffer B) (see example 2). Cultured *Clostridium difficile* cells were spiked into a 20% suspension of human feces in PBS-DOC/NP-40 in different concentrations, viz. $2.5 \times 10^5$ cells (solid lines), $2.5 \times 10^4$ cells (dotted lines), and $2.5 \times 10^3$ cells (solid lines with triangles). Lysis Buffer A or Lysis Buffer B were added to the feces suspensions together with 5000 cps of a plasmid DNA containing a cloned fragment of the *Bacillus subtilis* comK gene. Nucleic acid was extracted from the lysates and analyzed by real-time PCR amplification. (A) *Clostridium difficile* genomic DNA extracted with Lysis Buffer A; (B) *Clostridium difficile* genomic DNA extracted with Lysis Buffer B; (C) *Bacillus subtilis* plasmid DNA extracted with Lysis Buffer A; (D) *Bacillus subtilis* plasmid DNA extracted with Lysis Buffer B.

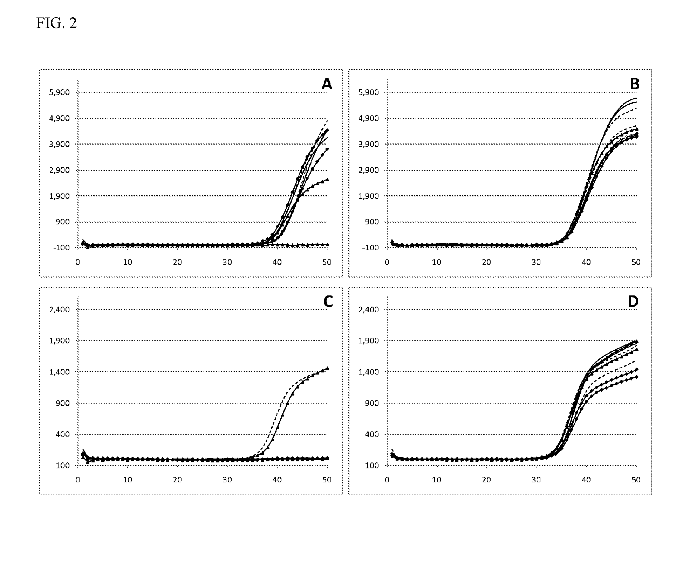

Fig. 2 PCR analysis of nucleic acid extracted from human feces samples spiked with *Clostridium difficile* tcdB plasmid DNA and *Bacillus subtilis* comK plasmid DNA using a lysis buffer without betaine (Lysis Buffer A) and a lysis buffer supplemented with 1.0 M betaine (Lysis Buffer B) (see example 3). Plasmid DNAs were spiked into a 20% suspension of human feces in PBS-DOC/NP-40 at 5000 cps per extraction. Lysis Buffer A or Lysis Buffer B were added to the feces suspensions and nucleic acid was extracted from the lysates followed by real-time PCR amplification with multiplex mixtures Mix 1 (solid lines with triangles), Mix 4 (solid lines with rectangles), Mix 5 (dashed lines), and Mix 7 (solid lines). (A) *Clostridium difficile* plasmid DNA extracted with Lysis Buffer A; (B) *Clostridium difficile* plasmid DNA extracted with Lysis Buffer B; (C) *Bacillus subtilis* plasmid DNA extracted with Lysis Buffer A; (D) *Bacillus subtilis* plasmid DNA extracted with Lysis Buffer B.

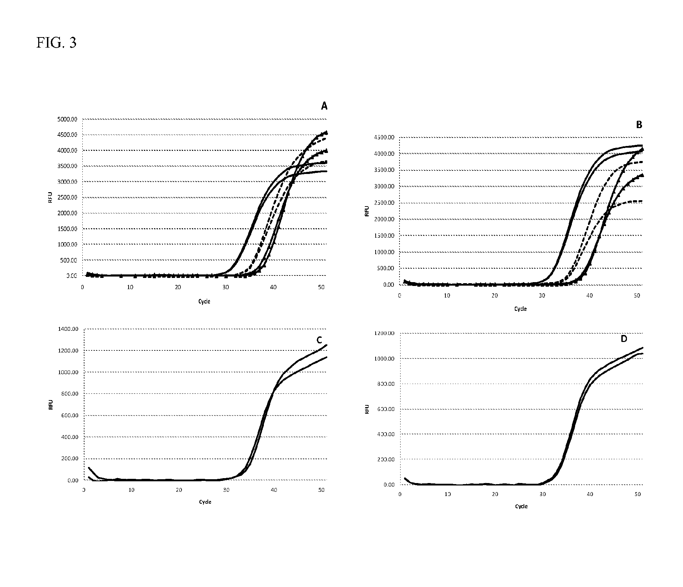

Fig. 3 PCR analysis of nucleic acid extracted from a human feces sample spiked with *Clostridium difficile* genomic DNA and *Bacillus subtilis* comK plasmid DNA using a lysis buffer without betaine (Lysis Buffer A) and a lysis buffer supplemented with 1.5 M betaine (Lysis Buffer B) (see example 4). Cultured *Clostridium difficile* cells were spiked into a 20% suspension of human feces in PBS-DOC/NP-40 in different concentrations, viz. $2.5 \times 10^5$ cells (solid lines), $2.5 \times 10^4$ cells (dotted lines), and $2.5 \times 10^3$ cells (solid lines with triangles). Lysis Buffer A or Lysis Buffer B were added to the feces suspensions together with 5000 cps of a plasmid DNA containing a cloned fragment of the *Bacillus subtilis* comK gene. Nucleic acid was extracted from the lysates and analyzed by real-time PCR amplification. (A) *Clostridium difficile* genomic DNA extracted with Lysis Buffer A; (B) *Clostridium difficile* genomic DNA extracted with Lysis Buffer B; (C) *Bacillus subtilis* plasmid DNA extracted with Lysis Buffer A; (D) *Bacillus subtilis* plasmid DNA extracted with Lysis Buffer B.

DETAILED DESCRIPTION OF THE EMBODIMENTS

The present invention relates to a buffer comprising in aqueous solution:

- at least one chaotropic agent
- at least one betaine.

According to the present invention a buffer is a solution containing a buffer substance that is suitable to stabilize the pH value of that solution. Numerous buffer substances are well known in the art. A typical example for a buffer substance is Tris. In a particularly preferred embodiment the present invention relates to compositions and uses employing Tris.

According to the present invention a chaotropic agent is a compound which is able to disrupt the three dimensional structure of a macromolecule such as a protein or nucleic acid by interfering with stabilizing intramolecular interactions. Typical examples of chaotropic agents include urea, guanidinium hydrochloride, guanidinium isothiocyanate (Gua-SCN). Another example of a chaotropic agent is lithium perchlorate.

According to the present invention betaines are compounds comprising a positively charged group as well as a negatively charged group in their molecular structure wherein hydrogen migration cannot compensate these charges. Preferred betaines include trimethylglycine and sultaine.

According to the present invention an aqueous solution is a solution formed from solid and liquid components, wherein water is the most abundant liquid component. Correspondingly, in non-aqueous solutions a non-aqueous component is the most abundant liquid component.

In a preferred embodiment of the present invention the buffers of the invention comprise:

- a chaotropic agent selected from the group consisting of:

    urea, guanidinium hydrochloride, guanidinium isothiocyanate (Gua-SCN), lithium perchlorate,

- a betaine selected from the group consisting of:

    trimethylglycine, sultaine.

In a particularly preferred embodiment of the present invention the buffers of the invention comprise:

- guanidinium isothiocyanate (Gua-SCN),
- trimethylglycine.

In a preferred embodiment of the present invention the buffers of the invention comprise additionally, at least one of the following components:

- a surfactant
- a chelating agent for metal ions.

According to the present invention a surfactant is an agent that reduces the surface tension of a liquid. Numerous anionic, cationic, non-ionic, and zwitterionic surfactants (categorized according to their charge) are well known in the art amongst which are a large number of surfactants compatible with protocols for the extraction and subsequent analysis of nucleic acids. In a preferred embodiment the invention relates to compositions and uses employing non-ionic surfactants. A typical example for a non-ionic surfactant is Triton X-100.

According to the present invention a chelating agent for metal ions is a compound that is capable of forming a chelate-complex with a metal ion, thus preventing its interaction in processes resulting in the degradation of macromolecules and in particular nucleic acids. Numerous chelating agents for metal ions are well known in the art. Typical examples are EDTA and EGTA. In a particularly preferred embodiment the present invention relates to compositions and uses employing EDTA.

In one aspect the present invention relates to compositions that contain at least one compound in a concentration that is close to its saturation concentration. In this context saturation concentration denotes the maximum concentration of a compound that can be obtained by dissolving that compound in a solution at a specific temperature. As the present invention relates in one aspect to increasing the maximal concentration obtainable by dissolving a compound in a solution by the addition of a betaine the saturation concentration for the purpose of the present invention is defined as the saturation concentration obtained in the absence of betaine additive. Therefore, in a preferred embodiment the present invention relates to buffers, wherein the concentration of the chaotropic agent ($c_{ca}$) and the concentration of the betaine ($c_{bet}$) are selected from the group of:

$$[(c_{sat} - 1\ M) > c_{ca} > (c_{sat} - 2\ M)] \text{ and } [2.5\ M > c_{bet} > 1.5\ M],$$

$$[(c_{sat} - 0.5\ M) > c_{ca} > (c_{sat} - 1\ M)] \text{ and } [2.5\ M > c_{bet} > 1.5\ M],$$

$$[(c_{sat} - 0.3 \text{ M}) > c_{ca} > (c_{sat} - 0.5 \text{ M})] \text{ and } [2.5 \text{ M} > c_{bet} > 1.5 \text{ M}],$$

$$[(c_{sat} - 0.1 \text{ M}) > c_{ca} > (c_{sat} - 0.3 \text{ M})] \text{ and } [2.5 \text{ M} > c_{bet} > 1.5 \text{ M}],$$

$$[c_{sat} > c_{ca} > (c_{sat} - 0.1 \text{ M})] \text{ and } [2.5 \text{ M} > c_{bet} > 1.5 \text{ M}],$$

$$[c_{ca} = c_{sat}] \text{ and } [2.5 \text{ M} > c_{bet} > 1.5 \text{ M}],$$

$$[(c_{sat} + 0.1 \text{ M}) > c_{ca} > c_{sat}] \text{ and } [2.5 \text{ M} > c_{bet} > 1.5 \text{ M}],$$

$$[(c_{sat} + 0.3 \text{ M}) > c_{ca} > (c_{sat} + 0.1 \text{ M})] \text{ and } [2.5 \text{ M} > c_{bet} > 1.5 \text{ M}],$$

$$[(c_{sat} + 0.5 \text{ M}) > c_{ca} > (c_{sat} + 0.3 \text{ M})] \text{ and } [2.5 \text{ M} > c_{bet} > 1.5 \text{ M}],$$

$$[(c_{sat} + 1 \text{ M}) > c_{ca} > (c_{sat} + 0.5 \text{ M})] \text{ and } [2.5 \text{ M} > c_{bet} > 1.5 \text{ M}],$$

$$[(c_{sat} + 2 \text{ M}) > c_{ca} > (c_{sat} + 1 \text{ M})] \text{ and } [2.5 \text{ M} > c_{bet} > 1.5 \text{ M}],$$

$$[(c_{sat} - 1 \text{ M}) > c_{ca} > (c_{sat} - 2 \text{ M})] \text{ and } [1.5 \text{ M} > c_{bet} > 1 \text{ M}],$$

$$[(c_{sat} - 0.5 \text{ M}) > c_{ca} > (c_{sat} - 1 \text{ M})] \text{ and } [1.5 \text{ M} > c_{bet} > 1 \text{ M}],$$

$$[(c_{sat} - 0.3 \text{ M}) > c_{ca} > (c_{sat} - 0.5 \text{ M})] \text{ and } [1.5 \text{ M} > c_{bet} > 1 \text{ M}],$$

$$[(c_{sat} - 0.1 \text{ M}) > c_{ca} > (c_{sat} - 0.3 \text{ M})] \text{ and } [1.5 \text{ M} > c_{bet} > 1 \text{ M}],$$

$$[c_{sat} > c_{ca} > (c_{sat} - 0.1 \text{ M})] \text{ and } [1.5 \text{ M} > c_{bet} > 1 \text{ M}],$$

$$[c_{ca} = c_{sat}] \text{ and } [1.5 \text{ M} > c_{bet} > 1 \text{ M}],$$

$[(c_{sat} + 0.1\ M) > c_{ca} > c_{sat}]$ and $[1.5\ M > c_{bet} > 1\ M]$,

$[(c_{sat} + 0.3\ M) > c_{ca} > (c_{sat} + 0.1\ M)]$ and $[1.5\ M > c_{bet} > 1\ M]$,

$[(c_{sat} + 0.5\ M) > c_{ca} > (c_{sat} + 0.3\ M)]$ and $[1.5\ M > c_{bet} > 1\ M]$,

$[(c_{sat} + 1\ M) > c_{ca} > (c_{sat} + 0.5\ M)]$ and $[1.5\ M > c_{bet} > 1\ M]$,

$[(c_{sat} + 2\ M) > c_{ca} > (c_{sat} + 1\ M)]$ and $[1.5\ M > c_{bet} > 1\ M]$,

$[(c_{sat} - 1\ M) > c_{ca} > (c_{sat} - 2\ M)]$ and $[1\ M > c_{bet} > 0.5\ M]$,

$[(c_{sat} - 0.5\ M) > c_{ca} > (c_{sat} - 1\ M)]$ and $[1\ M > c_{bet} > 0.5\ M]$,

$[(c_{sat} - 0.3\ M) > c_{ca} > (c_{sat} - 0.5\ M)]$ and $[1\ M > c_{bet} > 0.5\ M]$,

$[(c_{sat} - 0.1\ M) > c_{ca} > (c_{sat} - 0.3\ M)]$ and $[1\ M > c_{bet} > 0.5\ M]$,

$[c_{sat} > c_{ca} > (c_{sat} - 0.1\ M)]$ and $[1\ M > c_{bet} > 0.5\ M]$,

$[c_{ca} = c_{sat}]$ and $[1\ M > c_{bet} > 0.5\ M]$,

$[(c_{sat} + 0.1\ M) > c_{ca} > c_{sat}]$ and $[1\ M > c_{bet} > 0.5\ M]$,

$[(c_{sat} + 0.3\ M) > c_{ca} > (c_{sat} + 0.1\ M)]$ and $[1\ M > c_{bet} > 0.5\ M]$,

$[(c_{sat} + 0.5\ M) > c_{ca} > (c_{sat} + 0.3\ M)]$ and $[1\ M > c_{bet} > 0.5\ M]$,

$[(c_{sat} + 1\ M) > c_{ca} > (c_{sat} + 0.5\ M)]$ and $[1\ M > c_{bet} > 0.5\ M]$,

$$[(c_{sat} + 2\ M) > c_{ca} > (c_{sat} + 1\ M)] \text{ and } [1\ M > c_{bet} > 0.5\ M],$$

$$[(c_{sat} - 1\ M) > c_{ca} > (c_{sat} - 2\ M)] \text{ and } [0.5\ M > c_{bet}],$$

$$[(c_{sat} - 0.5\ M) > c_{ca} > (c_{sat} - 1\ M)] \text{ and } [0.5\ M > c_{bet}],$$

$$[(c_{sat} - 0.3\ M) > c_{ca} > (c_{sat} - 0.5\ M)] \text{ and } [0.5\ M > c_{bet}],$$

$$[(c_{sat} - 0.1\ M) > c_{ca} > (c_{sat} - 0.3\ M)] \text{ and } [0.5\ M > c_{bet}],$$

$$[c_{sat} > c_{ca} > (c_{sat} - 0.1\ M)] \text{ and } [0.5\ M > c_{bet}],$$

$$[c_{ca} = c_{sat}] \text{ and } [0.5\ M > c_{bet}],$$

$$[(c_{sat} + 0.1\ M) > c_{ca} > c_{sat}] \text{ and } [0.5\ M > c_{bet}],$$

$$[(c_{sat} + 0.3\ M) > c_{ca} > (c_{sat} + 0.1\ M)] \text{ and } [0.5\ M > c_{bet}],$$

$$[(c_{sat} + 0.5\ M) > c_{ca} > (c_{sat} + 0.3\ M)] \text{ and } [0.5\ M > c_{bet}],$$

$$[(c_{sat} + 1\ M) > c_{ca} > (c_{sat} + 0.5\ M)] \text{ and } [0.5\ M > c_{bet}],$$

$$[(c_{sat} + 2\ M) > c_{ca} > (c_{sat} + 1\ M)] \text{ and } [0.5\ M > c_{bet}],$$

wherein $C_{sat}$ is defined as the saturation concentration of the chaotropic agent in the corresponding solution at 25 °C if no betaine is added.

A simple experiment can be performed in order to test if a combination of concentrations chosen for chaotropic agent and betaine is suitable to stabilize a given solution with respect to crystal formation, e.g. during storage: A solution containing chaotropic agent and betaine at the concentrations chosen is prepared and subjected to the temperature conditions expected during storage (e.g. 2 °C to 8 °C as in a refrigerator), for a prolonged period of time, e.g. for 24 hours or one week. Subsequently, the solution is examined with respect to crystal formation, e.g. by visual inspection. If no crystals are found the concentrations chosen are suitable to sufficiently stabilize the solution (cf. example 1).

A simple experiment can be performed in order to test if a concentration chosen for a betaine in a lysis and extraction solution containing a chaotropic agent is suitable for improving the yields of extracted nucleic acids and/or is suitable for reducing the inhibitory and/or disturbing effect of the chaotropic agent on subsequent nucleic acid amplification reactions: A solution containing chaotropic agent and betaine at the concentrations chosen is prepared, combined with a nucleic acid sample and, subsequently, subjected to a nucleic acid amplification reaction (e.g. PCR). Alternatively, the sample containing the nucleic acid is subjected to a lysis and extraction protocol of interest using a lysis and extraction solution containing chaotropic agent and betaine at the concentrations chosen. The nucleic acid extracted thereby is then used for the amplification reaction. The experiment is performed with different betaine concentrations and the results of the nucleic acid amplification are compared thus yielding the most suitable betaine concentration. In the context of quantitative real-time PCR for example the most suitable betaine concentration may be defined as the lowest corresponding $C_t$-value for the sample.

In another embodiment of the present invention the buffers of the invention comprise in aqueous solution:

- 4.65 M to 4.85 M, preferably 4.75 M guanidinium isothiocyanate (Gua-SCN)
- 1.35 M to 1.65 M, preferably 1.5 M trimethylglycine.

The corresponding buffers combine the beneficial effect of the betaine additive, i.e. inhibition of the crystallization of guanidinium isothiocyanate at temperatures typical for storage in a refrigerator (e.g. 2°C to 8°C) with improved yields of extracted nucleic acids and/or significantly reduced inhibition and/or disturbance of subsequent amplification reactions (e.g. PCR) performed with the extracted nucleic acids. This was verified experimentally, the result is displayed in Fig. 3 (see example 4).

In another embodiment of the present invention the buffers of the invention comprise in aqueous solution:

- 5.15 M to 5.35 M, preferably 5.25 M guanidinium isothiocyanate (Gua-SCN) and
- 0.9 M to 1.1 M, preferably 1 M trimethylglycine.

These buffers are not stable towards crystallization of guanidinium isothiocyanate at temperatures typical for storage in a refrigerator (e.g. 2°C to 8°C), however, they result in high yields of extracted nucleic acids and/or very significantly reduced inhibition and/or disturbance of subsequent amplification reactions (e.g. PCR) performed with the extracted nucleic acids. This was verified experimentally, the result is displayed in Fig. 2 (see example 3).

In another embodiment the present invention further comprises a kit comprising, in packaged combination, at least one container containing a buffer of the invention and at least one container containing some or all of the reagents necessary for the amplification of nucleic acids. In a particularly preferred embodiment reagents required for nucleic acid amplification comprise at least one of the following: an oligonucleotide primer pair or a plurality of different oligonucleotide primer pairs, nucleotides, a polymerase, a label that allows to monitor the amplification process, a buffer for the amplification reaction.

In another embodiment the present invention further comprises a cartridge containing a buffer according to the invention. According to the present invention a cartridge is suitable to be used by an automated bioanalytical instrument and is further suitable for storing liquid solutions and sample material so that liquid solutions and sample material in one cartridge can temporarily be stored while the liquid solutions and sample material in another cartridge are being processed by the automated bioanalytical instrument. In a preferred embodiment the present invention relates to cartridges which are built in such a way that it is difficult or impossible to assess from the outside if precipitate is present in the cartridge. In another preferred embodiment the present invention relates to cartridges which are built in such a way that it is difficult or impossible to take measures suitable to re-dissolve precipitate present in the cartridge.

In another embodiment the present invention further comprises a process for the lysis of sample material and/or extraction and/or analysis of nucleic acids from sample material comprising the step:

Exposing the sample material to a buffer according to the invention.

The sample material containing the nucleic acid to be extracted contains in addition to the nucleic acid at least one of the following: proteins, lipids, carbohydrates. In a preferred embodiment the sample material is of biological origin. Typical sample materials include for example: cells, viruses, tissue, body fluids.

In a preferred embodiment of the present invention the nucleic acid to be analyzed is DNA. In a particularly preferred embodiment of the present invention the nucleic acid to be analyzed is plasmid DNA.

A large number of processes for the lysis of sample material and/or extraction of nucleic acids from different sample materials are known in the art. A number of such processes employ buffers containing chaotropic agents. During the extraction procedure from sample materials of biological origin the chaotropic agent effects denaturation of biological macromolecules, the disruption of non-covalent bonds and inhibition of enzymes that would otherwise degrade the nucleic acids. In addition to exposing the sample material to the lysis composition, typically, the process of lysis and/or extraction can also involve some kind of mechanical disruption, e.g. mechanical blender, glass beads, grinding, French pressure cell, homogenizer, sonication or freeze-thaw-cycles. A typical process for the lysis of sample material and extraction of nucleic acids thereof comprises the steps: (i) exposing the sample material to a buffer according to the invention, (ii) specific binding of nucleic acids to a suitable substance in a buffer according to the invention, (iii) washing of the substance with bound nucleic acids with one or more solutions suitable for this step, (iv) elution of the nucleic acids from the substance with a solution suitable for this step.

According to the present invention processes for the analysis of sample material are preferably processes involving the amplification of nucleic acids contained in the sample material. Analysis-methods involving the amplification of nucleic acids (e.g. by polymerase chain reaction (PCR), nucleic acid sequence-based amplification (NASBA) or ligase chain reaction (LCR)) are well known in the art. In a preferred embodiment of the present invention the analysis of sample material involves a quantitative real time PCR process. Such processes are well known in the art. In another preferred embodiment of the present invention the nucleic acid that is analyzed is DNA. In a particularly preferred embodiment of the present invention the nucleic acid that is analyzed is plasmid DNA.

In a preferred embodiment of the present invention the lysis and extraction protocol followed by nucleic acid amplification is performed as follows:

A lysis buffer is prepared and used to extract nucleic acids from sample material. Typically, two volumes of lysis buffer are added to one volume of sample and the resulting lysates are incubated for 10 minutes to establish complete lysis. In a following step, the lysate is applied to a spin column containing a silica membrane. The spin column is centrifuged and the flow-through is discarded. Next, the silica membrane is washed with a first wash buffer containing a high concentration of chaotropic salt to remove residual unbound substances and subsequently with a wash buffer containing a high concentration of ethanol to remove the chaotropic salts while keeping the nucleic acid bound to the silica membrane. After removal of the ethanol remnants by an extra centrifugation step, bound nucleic acids are eluted from the silica membrane by applying a pre-warmed low salt buffer to the spin column followed by a short incubation prior to centrifugation. The flow-through of the elution step is collected in a clean tube and subsequently used for PCR analysis. For the amplification of specific target DNA segments, an amount of the eluate with the nucleic acids extracted from the sample is mixed with a buffer containing the ingredients necessary for PCR amplification (deoxyribonucleotides, $MgCl_2$, Tris-HCl buffer, KCl, Taq DNA polymerase) and oligonucleotide primers and probes derived from the target DNA primary structure. The PCR reaction mixture is denatured at an elevated temperature to melt out any double-stranded DNA strains. Subsequently, the target DNA segments are amplified by alternately incubating the PCR reaction mixture at a high temperature (typically 95°C) for denaturation and at a lower temperature (typically 60°C) for primer and probe annealing and primer extension. Probe signals are monitored over time and are measured during each annealing step.

In another embodiment the present invention further comprises the use of a betaine as an additive for a buffer that is suitable for the extraction of nucleic acids from sample material, wherein the sample material, in addition to the nucleic acids, contains at least one of the following components: proteins, lipids, carbohydrates.

In another embodiment the present invention further comprises the use of a betaine for improving lysis and extraction solutions containing a chaotropic agent, wherein the use of these solutions in the course of the lysis and extraction procedure results in higher yields of extracted nucleic acids and/or in less inhibition and/or disturbance of subsequent nucleic acid amplification reactions performed with the extracted nucleic acids, than the use of lysis and extraction solutions containing no such betaine additives.

A large number of effects are known in the art that result in the reduction of extraction-yield and/or inhibition and/or disturbance of nucleic acid amplification reactions. Such effects can be caused by the presence of detrimental agents. People of skill in the art can readily identify such effects. In the context of quantitative real time PCR for example such an effect can be identified by comparing the curve displaying the temporal development of the signal correlated to nucleic acid amplification for a sample containing a potentially detrimental agent to that of a sample without such an agent. In a preferred embodiment of the present invention such an effect that results in the reduction of extraction-yield and/or inhibition and/or disturbance of nucleic acid amplification reactions is characterized by an increased $C_t$-value for the sample that is subject to this effect.

In another embodiment the present invention further comprises the use of a betaine for stabilizing a compound in a solution. According to the present invention stabilization of the compound in the solution is achieved if at constant temperature the saturation concentration of that compound can be increased by the addition of the betaine. The saturation concentration of a compound is the maximal concentration at which that compound can be dissolved at a specific temperature. The solution can be an aqueous solution or a non-aqueous solution. In a preferred embodiment the solution is an aqueous solution.

In a preferred embodiment of the present invention the compound to be stabilized in a solution is either an organic salt or a chaotropic agent. According to the present invention an organic salt is a salt of an organic cation. Typical organic cations are guanidinium and ammonium.

In another preferred embodiment of the present invention the compound to be stabilized in a solution is selected from the group of:

urea, guanidinium hydrochloride, guanidinium isothiocyanate (Gua-SCN) and lithium perchlorate

In another preferred embodiment of the present invention, the betaine for stabilizing the compound in solution is selected from the group of:

trimethylglycine, sultaine.

EXAMPLES

Example 1:

Aqueous solutions comprising different concentrations of Guanidinium isothiocyanate (Gua-SCN) and Triton X-100, EDTA as well as Tris-HCl (pH 6.4) were prepared at room temperature and trimethylglycine was added in different concentrations. Subsequently, the solutions were cooled down to a temperature of 2 °C and stored at that temperature in a refrigerator for one week. After that crystal formation was examined by visual inspection. Table 1 contains a summary of the results: +++ = formation of large amounts of crystals, ++ = formation of intermediate amounts of crystals, + = formation of low amounts of crystals, 0 = no formation of crystals. The effect of the addition of trimethylglycine on the formation of crystals is clearly visible. Adding 1.5 M trimethylglycine to a solution of 4.7 M Gua-SCN or 2 M trimethylglycine to a solution of 5.25 M Gua-SCN prevents crystal formation.

Table 1: Effect of the addition of trimethylglycine on crystal formation at 2 °C.

| Gua-SCN | Triton X-100 | EDTA | Tris-HCl (pH 6.4) | Trimethylglycine | |
|---|---|---|---|---|---|
| [M] | [wt%] | [mM] | [mM] | [M] | Crystal formation |
| 4.7 | 1.3 | 20 | 50 | 0 | ++ |
| 4.7 | 1.3 | 20 | 50 | 0.5 | + |
| 4.7 | 1.3 | 20 | 50 | 1 | + |
| 4.7 | 1.3 | 20 | 50 | 1.5 | 0 |
| 4.7 | 1.3 | 20 | 50 | 2 | 0 |
| 5.25 | 1.3 | 20 | 50 | 0 | ++ |
| 5.25 | 1.3 | 20 | 50 | 0.5 | + |
| 5.25 | 1.3 | 20 | 50 | 1 | + |
| 5.25 | 1.3 | 20 | 50 | 1.5 | + |
| 5.25 | 1.3 | 20 | 50 | 2 | 0 |

Example 2:

The following experiment was performed in order to show that adding trimethylglycine does not negatively affect the overall assay. Two lysis buffers were prepared. One of these buffers (Lysis Buffer A) consisted of 5.25 M Gua-SCN, 50 mM Tris-HCl (pH 6.4), 20 mM EDTA, and 1.3 % (w/v) Triton X-100. The other lysis buffer (Lysis Buffer B) had exactly the same composition with the exception that it additionally contained 1.0 M trimethylglycine. These lysis buffers were used to extract nucleic acid from difficult sample matrices known to contain high concentrations of inhibitory factors for PCR amplification and high levels of background DNA competing with the target DNA of interest for binding places on the silica moiety. A 20 % (w/v) suspension of a human feces sample was prepared in Phosphate Buffered Saline (PBS) solution supplemented with 1.0 % deoxycholate (DOC) and 1.0 % Nonidet-P40 (NP-40). To 400 μl of this feces suspension (equivalent to 80 mgr feces) 800 μl of either Lysis Buffer A (without trimethylglycine) or Lysis Buffer B (with 1.0 M trimethylglycine) were added. To these lysates different amounts of a culture of *Clostridium difficile* cells of strain ATCC 9689T were added containing about $2.5 \times 10^5$, $2.5 \times 10^4$, or $2.5 \times 10^3$ cells. Additionally, 5000 copies of a plasmid DNA containing a cloned fragment of the *Bacillus subtilis* comK gene were spiked into the lysate as a so-called Sample Processing Control (SPC) to monitor extraction and PCR analysis. Upon centrifugation of the resulting suspension during 2 minutes to remove any undissolved feces components, the cleared lysate was applied to a NucleoSpin Blood Column (Macherey-Nagel, Düren, Germany) in two portions of 550 µl and each portion was incubated during 1 minute at room temperature prior to centrifugation through the silica membrane in the spin column. After that the spin column was centrifuged and the flow-through was discarded. Next, the silica membrane was washed with a first wash buffer containing a high concentration of chaotropic salt to remove residual unbound substances (600 µl BW Buffer (Macherey-Nagel, Düren, Germany)) and subsequently with a wash buffer containing a high concentration of ethanol to remove the chaotropic salts while still keeping the nucleic acid bound to the silica membrane (600 µl B5 Buffer (Macherey-Nagel, Duren, Germany)) followed by centrifugation during 1 minute for each of the wash buffers. After the final washing step, residual ethanol was removed from the silica membrane by an extra centrifugation step of 3 minutes. Finally, bound nucleic acids were eluted from the silica membrane by applying two aliquots of 75 µl of BE Buffer (Macherey-Nagel, Düren, Germany) to the spin columns that were pre-warmed to 70°C followed by a short incubation (2 minutes at room temperature) prior to centrifugation (1 minute). Flow-through of the elution step was collected in a clean tube and subsequently used for PCR analysis. Upon elution, the two eluate aliquots were pooled and immediately used for PCR analysis. For the PCR reactions, 10 µl of the nucleic acid extracts prepared with either Lysis Buffer A or Lysis Buffer B, were mixed with 12.5 µl LightCycler® 480 Probes Master (Roche Diagnostics GmbH, Mannheim, Germany) mastermix and 0.5 µl of a 90 mM $MgCl_2$ solution. To these mixtures, 2 µl of a multiplex PCR mix of primers and Taqman probes were added targeting two regions on the tcdB gene of *Clostridium difficile* and the cloned *Bacillus subtilis* comK fragment in the plasmid DNA that was spiked into each sample as the SPC. Taqman probes for the individual PCR targets were labeled with fluorophores FAM for comK and Yakima Yellow and ATT0647N for the tcdB gene targets. PCR reaction mixtures were processed in a Bio-Rad CFX96 system (Bio-Rad Laboratories, Veenendaal, The Netherlands). Denaturation was performed at 95°C during 10 minutes and was followed by 50 cycles, each consisting of 15 seconds denaturation at 95°C and 60 seconds annealing/extension at 60°C. Fluorescence was monitored over time and measured at the end of each annealing/extension step. Real-time PCR curves that were obtained for the different PCR targets in the different samples are shown in Figure 1. From the figure it can be seen that for the tcdB targets that were amplified from the *Clostridium difficile* genomic DNA that was extracted from the 20% human feces suspensions in PBS/DOC-NP-40, no difference is observed between the extractions for which Lysis Buffer A or Lysis Buffer B were used. For both lysis buffers, all PCR reactions are positive for all of the input levels of cultured *Clostridium difficile* cells and Ct values are comparable for the different input levels and the different amounts of feces that were used. Consequently, the addition of trimethylglycine to the lysis buffer has no negative effects on the extraction of bacterial genomic DNA from a human feces sample. However, for the comK plasmid DNA a clear difference was observed between Lysis Buffers A and B. For Lysis Buffer A, not all PCR reactions revealed a positive result and some variation in the Ct values for the individual PCR curves was observed despite the fact that each sample was spiked with the same amount of comK plasmid DNA. For Lysis Buffer B, all PCR reactions were positive and Ct values were much more consistent as for Lysis Buffer A. The example shows that trimethylglycine addition has no negative effect on nucleic acid extraction and, unexpectedly, reveals even better results for plasmid DNA. Therefore, addition of 1.0 M of trimethylglycine to the lysis buffer used in a silica-based nucleic acid extraction procedure is beneficial for the extraction of plasmid DNA spiked into a human feces sample.

Example 3:

The following experiment was performed in order to show that adding trimethylglycine improves nucleic acid yield and/or reduces inhibitory and/or disturbing effects resulting from chaotropic agents in the lysis and extraction buffer. Lysis Buffer A (as defined in example 2) and Lysis Buffer B (as defined in example 2) were used to extract nucleic acid from a 20 % human feces suspension in PBS/DOC-NP-40 essentially as described for example 2 with the only difference that the tcdB target was not spiked into the lysates as genomic DNA from cultured *Clostridium difficile* cells but as plasmid DNA containing the target PCR segment as a cloned fragment. Similar to the *Bacillus subtilis* comK plasmid DNA, 5000 copies of the tcdB fragment plasmid DNA were spiked into each lysate. Subsequently, nucleic acid extraction and PCR analysis were performed essentially as described in example 2. For PCR analysis, each of the eluates now was amplified with four different multiplex PCR mixtures. Each of the mixtures contained the primers and probes for three PCR targets, one of which was the tcdB gene PCR target and another one was the *Bacillus subtilis* comK PCR target. The third PCR target in each of the multiplex PCR mixtures was variable. Results of this PCR analysis are depicted in Figure 2. For all three PCR targets for which the results are shown and that were amplified from the corresponding plasmid DNAs as extracted from the feces lysates prepared with either Lysis Buffer A or Lysis Buffer B, it was observed that better results were obtained for Lysis Buffer B, i.e. for the lysis buffer containing 1.0 M trimethylglycine. For this lysis buffer, all PCR reactions revealed a positive result and Ct values were lower and more consistent as for their counterparts obtained with Lysis Buffer A. In addition, not all PCR reactions revealed a positive result for the eluates obtained with Lysis Buffer A.

Example 4:

The experiment performed was exactly the same as in example 2, but performed with different buffers. Lysis Buffer A consisted of 4.75 M Gua-SCN, 50 mM Tris-HCl (pH 6.4), 20 mM EDTA, and 1.3 % (w/v) Triton X-100. The other lysis buffer (Lysis Buffer B) had exactly the same composition with the exception that it additionally contained 1.5 M trimethylglycine. Results of the PCR analysis are depicted in Figure 3.

**Claims**

1. Buffer comprising in aqueous solution:

   at least one chaotropic agent
   at least one betaine.

2. Buffer according to claim 1, wherein:

   the chaotropic agent is selected from the group consisting of:

   urea, guanidinium hydrochloride, guanidinium isothiocyanate (Gua-SCN), lithium perchlorate
   the betaine is selected from the group consisting of:

   trimethylglycine, sultaine.

3. Buffer according to claims 1 to 2, additionally comprising at least one of the following components:

   a surfactant
   a chelating agent for metal ions

4. Buffer according to claims 1 to 3, wherein the concentration of the chaotropic agent ($C_{ca}$) and the concentration of the betaine ($C_{bet}$) are selected from the group of:

$$[(c_{sat} - 1\ M) > c_{ca} > (c_{sat} - 2\ M)] \text{ and } [2.5\ M > c_{bet} > 1.5\ M],$$

$$[(c_{sat} - 0.5\ M) > c_{ca} > (c_{sat} - 1\ M)] \text{ and } [2.5\ M > c_{bet} > 1.5\ M],$$

$$[(c_{sat} - 0.3\ M) > c_{ca} > (c_{sat} - 0.5\ M)] \text{ and } [2.5\ M > c_{bet} > 1.5\ M],$$

$$[(c_{sat} - 0.1\ M) > c_{ca} > (c_{sat} - 0.3\ M)] \text{ and } [2.5\ M > c_{bet} > 1.5\ M],$$

$$[c_{sat} > c_{ca} > (c_{sat} - 0.1\ M)] \text{ and } [2.5\ M > c_{bet} > 1.5\ M],$$

$$[c_{ca} = c_{sat}] \text{ and } [2.5\ M > c_{bet} > 1.5\ M],$$

$$[(c_{sat} + 0.1\ M) > c_{ca} > c_{sat}] \text{ and } [2.5\ M > c_{bet} > 1.5\ M],$$

$[(c_{sat} + 0.3\ M) > c_{ca} > (c_{sat} + 0.1\ M)]$ and $[2.5\ M > c_{bet} > 1.5\ M]$,

$[(c_{sat} + 0.5\ M) > c_{ca} > (c_{sat} + 0.3\ M)]$ and $[2.5\ M > c_{bet} > 1.5\ M]$,

$[(c_{sat} + 1\ M) > c_{ca} > (c_{sat} + 0.5\ M)]$ and $[2.5\ M > c_{bet} > 1.5\ M]$,

$[(c_{sat} + 2\ M) > c_{ca} > (c_{sat} + 1\ M)]$ and $[2.5\ M > c_{bet} > 1.5\ M]$,

$[(c_{sat} - 1\ M) > c_{ca} > (c_{sat} - 2\ M)]$ and $[1.5\ M > c_{bet} > 1\ M]$,

$[(c_{sat} - 0.5\ M) > c_{ca} > (c_{sat} - 1\ M)]$ and $[1.5\ M > c_{bet} > 1\ M]$,

$[(c_{sat} - 0.3\ M) > c_{ca} > (c_{sat} - 0.5\ M)]$ and $[1.5\ M > c_{bet} > 1\ M]$,

$[(c_{sat} - 0.1\ M) > c_{ca} > (c_{sat} - 0.3\ M)]$ and $[1.5\ M > c_{bet} > 1\ M]$,

$[c_{sat} > c_{ca} > (c_{sat} - 0.1\ M)]$ and $[1.5\ M > c_{bet} > 1\ M]$,

$[c_{ca} = c_{sat}]$ and $[1.5\ M > c_{bet} > 1\ M]$,

$[(c_{sat} + 0.1\ M) > c_{ca} > c_{sat}]$ and $[1.5\ M > c_{bet} > 1\ M]$,

$[(c_{sat} + 0.3\ M) > c_{ca} > (c_{sat} + 0.1\ M)]$ and $[1.5\ M > c_{bet} > 1\ M]$,

$[(c_{sat} + 0.5\ M) > c_{ca} > (c_{sat} + 0.3\ M)]$ and $[1.5\ M > c_{bet} > 1\ M]$,

$[(c_{sat} + 1\ M) > c_{ca} > (c_{sat} + 0.5\ M)]$ and $[1.5\ M > c_{bet} > 1\ M]$,

$[(c_{sat} + 2\ M) > c_{ca} > (c_{sat} + 1\ M)]$ and $[1.5\ M > c_{bet} > 1\ M]$,

$$[(c_{sat} - 1\ M) > c_{ca} > (c_{sat} - 2\ M)]\ and\ [1\ M > c_{bet} > 0.5\ M],$$

$$[(c_{sat} - 0.5\ M) > c_{ca} > (c_{sat} - 1\ M)]\ and\ [1\ M > c_{bet} > 0.5\ M],$$

$$[(c_{sat} - 0.3\ M) > c_{ca} > (c_{sat} - 0.5\ M)]\ and\ [1\ M > c_{bet} > 0.5\ M],$$

$$[(c_{sat} - 0.1\ M) > c_{ca} > (c_{sat} - 0.3\ M)]\ and\ [1\ M > c_{bet} > 0.5\ M],$$

$$[c_{sat} > c_{ca} > (c_{sat} - 0.1\ M)]\ and\ [1\ M > c_{bet} > 0.5\ M],$$

$$[c_{ca} = c_{sat}]\ and\ [1\ M > c_{bet} > 0.5\ M],$$

$$[(c_{sat} + 0.1\ M) > c_{ca} > c_{sat}]\ and\ [1\ M > c_{bet} > 0.5\ M],$$

$$[(c_{sat} + 0.3\ M) > c_{ca} > (c_{sat} + 0.1\ M)]\ and\ [1\ M > c_{bet} > 0.5\ M],$$

$$[(c_{sat} + 0.5\ M) > c_{ca} > (c_{sat} + 0.3\ M)]\ and\ [1\ M > c_{bet} > 0.5\ M],$$

$$[(c_{sat} + 1\ M) > c_{ca} > (c_{sat} + 0.5\ M)]\ and\ [1\ M > c_{bet} > 0.5\ M],$$

$$[(c_{sat} + 2\ M) > c_{ca} > (c_{sat} + 1\ M)]\ and\ [1\ M > c_{bet} > 0.5\ M],$$

$$[(c_{sat} - 1\ M) > c_{ca} > (c_{sat} - 2\ M)]\ and\ [0.5\ M > c_{bet}],$$

$$[(c_{sat} - 0.5\ M) > c_{ca} > (c_{sat} - 1\ M)]\ and\ [0.5\ M > c_{bet}],$$

$$[(c_{sat} - 0.3\ M) > c_{ca} > (c_{sat} - 0.5\ M)]\ and\ [0.5\ M > c_{bet}],$$

$$[(c_{sat} - 0.1\ M) > c_{ca} > (c_{sat} - 0.3\ M)]\ and\ [0.5\ M > c_{bet}],$$

$$[c_{sat} > c_{ca} > (c_{sat} - 0.1\ M)]\ \text{and}\ [0.5\ M > c_{bet}],$$

$$[c_{ca} = c_{sat}]\ \text{and}\ [0.5\ M > c_{bet}],$$

$$[(c_{sat} + 0.1\ M) > c_{ca} > c_{sat}]\ \text{and}\ [0.5\ M > c_{bet}],$$

$$[(c_{sat} + 0.3\ M) > c_{ca} > (c_{sat} + 0.1\ M)]\ \text{and}\ [0.5\ M > c_{bet}],$$

$$[(c_{sat} + 0.5\ M) > c_{ca} > (c_{sat} + 0.3\ M)]\ \text{and}\ [0.5\ M > c_{bet}],$$

$$[(c_{sat} + 1\ M) > c_{ca} > (c_{sat} + 0.5\ M)]\ \text{and}\ [0.5\ M > c_{bet}],$$

$$[(c_{sat} + 2\ M) > c_{ca} > (c_{sat} + 1\ M)]\ \text{and}\ [0.5\ M > c_{bet}],$$

wherein $C_{sat}$ is defined as the saturation concentration of the chaotropic agent in the corresponding solution at 25 °C if no betaine is added.

5.  Buffer according to claims 1 to 4 comprising in aqueous solution:

    4.65 M to 4.85 M, preferably 4.75 M guanidinium isothiocyanate (Gua-SCN)
    1.35 M to 1.65 M, preferably 1.5 M trimethylglycine.

6.  Buffer according to claims 1 to 4 comprising in aqueous solution:

    5.15 M to 5.35 M, preferably 5.25 M guanidinium isothiocyanate (Gua-SCN) 0.9 M to 1.1 M, preferably 1 M trimethylglycine.

7.  A kit comprising, in packaged combination, at least one container containing a buffer according to claims 1 to 6 and at least one container containing some or all of the reagents necessary for the amplification of nucleic acids.

8.  Cartridge containing a buffer according to claims 1 to 6.

9.  Process for the lysis of sample material and/or extraction and/or analysis of nucleic acids from sample material comprising the step:

    Exposing the sample material to a buffer according to claims 1 to 6.

10. Process according to claim 9, wherein the nucleic acid to be analyzed is DNA, and preferably, wherein the nucleic acid to be analyzed is plasmid DNA.

11. Use of a betaine as an additive for a buffer, that is suitable for the extraction of nucleic acids from sample material, wherein the sample material, in addition to the nucleic acids, contains at least one of the following components: proteins, lipids, carbohydrates.

**12.** Use of a betaine for improving lysis and extraction solutions containing a chaotropic agent, wherein the use of these solutions in the course of the lysis and extraction procedure results in higher yields of extracted nucleic acids and/or in less inhibition and/or disturbance of subsequent nucleic acid amplification reactions performed with the extracted nucleic acids, than the use of lysis and extraction solutions containing no such betaine additives.

**13.** Use of a betaine for stabilizing a compound in a solution.

**14.** Use according to claim 13, wherein the compound is either an organic salt or a chaotropic agent.

**15.** Use according to claims 13 to 14, wherein the compound is selected from the group of:

urea, guanidinium hydrochloride, guanidinium isothiocyanate (Gua-SCN) or lithium perchlorate and,

wherein the betaine is selected from the group of:

Trimethylglycine, sultaine.

FIG. 1

FIG. 2

FIG. 3

Europäisches
Patentamt

European
Patent Office

Office européen
des brevets

## EUROPEAN SEARCH REPORT

Application Number

EP 10 16 4129

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/134470 A2 (3M INNOVATIVE PROPERTIES CO [US]; PARTHASARATHY RANJANI V [US]; LIU HS) 6 November 2008 (2008-11-06) * p.30, par.1; p.30, par.4-p.31, par.1 * | 1-15 | INV. G01N1/30 C12Q1/68 |
| X | WO 2009/085355 A2 (LONGHORN VACCINES & DIAGNOSTIC [US]; FISCHER GERALD W [US]; DAUM LUKE) 9 July 2009 (2009-07-09) * p.3, par.4; p.6, par.3-4; p.7, par.2; claims 1, 3 * | 1-15 | |
| X | WO 99/39000 A1 (AKZO NOBEL NV [NL]; GEMEN BOB VAN [NL]; SCHIJNDEL HARMANNUS BERNARDUS) 5 August 1999 (1999-08-05) * p.4, par.3 * | 1-15 | |
| X | CHINI E N ET AL: "The enhancement of Ca<2+> efflux from sarcoplasmic reticulum vesicles by urea" ARCHIVES OF BIOCHEMISTRY AND BIOPHYSICS, ACADEMIC PRESS, US LNKD-DOI:10.1016/0003-9861(92)90245-R, vol. 299, no. 1, 15 November 1992 (1992-11-15), pages 73-76, XP024762390 ISSN: 0003-9861 [retrieved on 1992-11-15] * table I * | 1-6,8 | TECHNICAL FIELDS SEARCHED (IPC)  G01N C12Q |
| X | WO 2008/002740 A2 (SIGMA ALDRICH CO [US]; MICHALIK STEVE S [US]; WEBER SCOTT A [US]; KREA) 3 January 2008 (2008-01-03) * par.0025; claim 1 * | 11 | |

-/--

The present search report has been drawn up for all claims

| Place of search | Date of completion of the search | Examiner |
|---|---|---|
| Munich | 29 June 2010 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 16 4129

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BATEMAN J B ET AL: "Dielectric properties of the system bovine albumin : urea : betaine in aqueous solution" PHYSICS IN MEDICINE AND BIOLOGY, TAYLOR AND FRANCIS LTD. LONDON, GB LNKD-DOI:10.1088/0031-9155/37/1/012, vol. 37, no. 1, 1 January 1992 (1992-01-01), pages 175-182, XP020021929 ISSN: 0031-9155 * p.175, par.2 * | 13-15 | |

TECHNICAL FIELDS
SEARCHED        (IPC)

The present search report has been drawn up for all claims

| Place of search | Date of completion of the search | Examiner |
|---|---|---|
| Munich | 29 June 2010 | Pellegrini, Paolo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 10 16 4129

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2010

| Patent document cited in search report | | Publication date | Patent family member(s) | | Publication date |
|---|---|---|---|---|---|
| WO 2008134470 | A2 | 06-11-2008 | EP | 2140001 A2 | 06-01-2010 |
| | | | US | 2010129878 A1 | 27-05-2010 |
| WO 2009085355 | A2 | 09-07-2009 | AU | 2008343745 A1 | 09-07-2009 |
| | | | CA | 2701168 A1 | 09-07-2009 |
| | | | EP | 2195466 A2 | 16-06-2010 |
| | | | US | 2009312285 A1 | 17-12-2009 |
| WO 9939000 | A1 | 05-08-1999 | AU | 759401 B2 | 17-04-2003 |
| | | | AU | 2621899 A | 16-08-1999 |
| | | | CA | 2317055 A1 | 05-08-1999 |
| | | | JP | 2002501759 T | 22-01-2002 |
| | | | US | 6465639 B1 | 15-10-2002 |
| | | | ZA | 9900493 A | 22-07-1999 |
| WO 2008002740 | A2 | 03-01-2008 | US | 2008003574 A1 | 03-01-2008 |
| | | | US | 2008003575 A1 | 03-01-2008 |

EPO FORM P0459

For more details about this annex : see Official Journal of the European Patent Office, No. 12/82